# EUROPEAN PATENT APPLICATION

(11) **EP 1 000 628 A1**
(43) Date of publication of application: **17.05.2000**
(21) Application number: 98402381.2
(22) Date of filing: 28.09.1998
(51) Int. Cl.: A61K 39/39, A61K 39/21, A61K 39/00, C12N 5/10

(54) **Use of antigenic complexes of HIV envelope and HLA class I antigens as HIV vaccine**

(71) Applicant: Fondation Mondiale Recherche et Prevention SIDA, 75732 Paris Cédex 15 (FR)
(72) Inventor: The designation of the inventor has not yet been filed
(74) Representative: Vialle-Presles, Marie José

(57) **Abstract**

Immunogenic compositions able to induce a protection against human immunodeficiency virus type 1 and type 2 (HIV-1 and HIV-2) and its applications.

Said compositions comprise:
. a composition essentially containing a multimolecular complex consisting essentially in at least an immunogenic fragment of the Env glycoprotein of an HIV and an HLA class I heavy chain (HC) or a fragment thereof, said elements being preferably non-covalently associated;
. a composition essentially containing particles containing, preferably at their surface, a multimolecular complex comprising essentially at least an immunogenic fragment of the Env glycoprotein of HIV and an HLA class I heavy chain or a fragment thereof or
. a composition containing a mixture of at least a fragment of HIV envelope glycoprotein and virion associated HLA class I heavy chains or a fragment thereof.

## Description

The invention relates to immunogenic compositions able to induce a protection against human immunodeficiency virus type 1 and type 2 (HIV-1 and HIV-2) and its applications.

It has been found that infection with human immunodeficiency virus type 1 and type 2 (HIV-1 and HIV-2) requires the interaction between the envelope glycoprotein surface unit (SU) gp120 and other co-factors or co-receptors. Although human immunodeficiency virus uses the T cell surface molecule CD4 (Klatzmann et al., *Nature*, 1984, **312**, 767-768 ; McDougal et al., *J. Immunol*., 1986, **137**, 2937-2944) as primary receptor, early studies suggested that CD4 was necessary but not sufficient to allow HIV entry and that other co-factors are required to permit envelope-mediated fusion of the viral and cellular membrane (Benkirane M. et al., **J. Virol.**, 1994, 6332-6339).

Indeed, successful viral entry into and infection of a cell has been found to require the presence of other molecules or co-factors (Clapham et al., *Nature*, 1997, **388**, 230-231).

Several members of the family of chemokines receptors were identified as co-factors or co-receptors needed for HIV entry. In general, T cell lines adapted strains of HIV-1 (TCLA) use the CXC receptor 4 (CXCR4) (Dynamics of HIV infection, Science and Medicine, 1998, 36-45) whereas macrophage tropic (M tropic) strains use the co-receptor CCR5. Although the majority of HIV-1 strains use either one of these two co-receptors, dual-tropic strains also have been identified as well as strains which use alternative co-receptors, including CCR3 and CCR2b.

Major histocompatibility complex of class II (MHC-II) are able to increase HIV infectivity: for instance, early events of viral entry are influenced by the acquisition by the virus of host-derived cell proteins like Major Histocompatibility Complex (MHC) class II glycoproteins (Cantin et al., *Blood*, **90**, 3, 1091-1100, 1997; Cantin et al., *J . Virol*. , **71**, 3, 1922-1930, 1997) and ICAM-1 (Rizzuto et al., *J. Virol.* **71**, 6, 4847-4851, 1997). MHC class I molecules are also incorporated within the virus (Arthur et al., *Science*, **258**, 1935-1938, 1992 and Frank et al., *AIDS*, **10**, 1611-1620, 1996); however, their role in viral entry is still unknown. A role of these molecules in HIV replication has been suggested by the observation that antibodies to HLA class I antigens can suppress HIV replication *in vitro* (Briant et al., *J. Virol*., **70**, 5213-5220, 1996; Corbeau et al., *Eur. J. Immunol*., **21**, 865-871, 1991; Corbeau et al., *J. Virol*., **64**, 4, 1459-1464, 1990; Arthur et al., *Science*, 1992, **258**, 1935-1938).

HLA class I molecules are trimeric complexes composed of a 45 kD polymorphic heavy chain (HC) consisting of three structural domains, α1, α2 and α3 non-covalently associated with a monomorphic 12 kDa light chain, β-2 microglobulin (β₂m). The antigenic peptide fits into the highly polymorphic α1-α2 regions that together comprise the peptide binding groove (Bjorkman, 1990). Other conformations such as heavy chains not associated with β₂m are also present at the cell membrane (Schnabl, 1990). These conformations are predominantly expressed by HLA-C encoded heavy chains (Zemmour, 1992).

It has been shown that the expression of different isoforms of HLA class I antigens influences the extend of HIV induced cell to cell fusion (De Santis et al., *AIDS Res. Hum. Retrovir*., 1996, **12**, 11 1031-1040).

In primate models of lentivirus infection antibodies directed against virionic HLA class I molecules can confer protection against infection (Stott et al., *Nature,* 1992; Chan et al., *J. Exp. Med*., 1992). This protection however, is limited to immunisation with xenogeneic MHC antigens (Human HLA immunising monkeys) and is not observed with allogeneic immunisation (monkeys MHC immunising monkeys) (Plyanskaya et at., *AIDS Res. Hum. Retrovir*., 1997). Thus, the suggested use of allogeneic immunisation as a possible strategy to HIV vaccination remains controversial (Luscher et al., *AIDS Res. Hum. retrovir*., **14**, 7, 541-544, 1998).

HIV infection down-modulates the surface expression of trimeric HLA class I molecules (Schwartz, 1996), thus allowing escape from CD8 cell-mediated immune surveillance (Collins, 1998). This effect depends on the presence of a functional *nef* gene since the Nef protein affects the intracellular sorting of HLA-A and B molecules.

In addition to be down-modulated by HIV Nef protein, HLA class I molecules are incorporated by HIV during the process of assembly of viral particles. The physiological relevance of this phenomenon is till unclear as is the underlying mechanism. Furthermore, it is not known whether specific interactions between viral proteins and HLA class I occur, nor if the pattern of HLA class I expression on HIV infected cells is influenced by their association to HIV viral particles budding form the cell membrane.

Cells devoid of cell surface HLA class I trimeric complexes are permissive to the replication of some TCLA (T cell line adapted) viruses thus suggesting that HLA class I molecules are not required for the generation of infective viral particles (Clapham, 1991).

However, the Inventors have now found, unexpectedly, that incorporation of HLA class I antigens and more specifically heavy chains of HLA class I anti-gens, is needed for the generation of infective viral particles. Moreover they found that anti-class I antibody alone could not inhibit cell-free infection whereas Env glycoprotein or a fragment thereof and HLA class I heavy chain may form a multimolecular complex able to induce an immune protection against HIV.

The subject of the present invention is an immunogenic composition, characterised in that it essentially contains a multimolecular complex consisting essentially in at least an immunogenic fragment of the Env glycoprotein of an HIV and an HLA class I heavy chain (HC) or fragments thereof, said elements being preferably non-covalently associated.

The subject of the present invention is also an immunogenic composition, characterised in that it essentially contains particles containing preferably at their surface, a multimolecular complex comprising essentially at least an immunogenic fragment of the Env glycoprotein of HIV and an HLA class I heavy chain or a fragment thereof.

According to an advantageous embodiment of said composition, said particles are selected in the group consisting in viral particles and transfected cells.

According to an advantageous arrangement of this embodiment, said viral particles are preferably selected in the group consisting in attenuated viruses, recombinant attenuated viral particles and purified pseudoparticles. The viral particles are preferably selected among MVA (modified virus Ankara) vaccinia virus, adenovirus, canarypox, BCG, poliovirus, chickenpox, smallpox or baculovirus. More preferably, attenuated MVA vaccinia expressing HIV Env (with or without gag pol) may be used.

According to another an advantageous arrangement of this embodiment, said transfected cells are preferably selected in the group consisting in human or non-human cells, more preferably in the group consisting in B cells (.221 cells, DAUDI cells, for instance) and CHO cells.

The subject of the present invention is also a composition consisting in a mixture of at least a fragment of HIV envelope glycoprotein and virion associated HLA class I heavy chains or a fragment thereof.

According to an advantageous embodiment of all the compositions according to the invention, said HIV Env glycoprotein is selected in the group consisting of gp120, gp160 or immunogenic fragments thereof either from HIV-1 or HIV-2 ; said gp120 or gp160 may be recombinant envelope antigens.

According to another advantageous embodiment of all the composition according to the invention, said HLA class I heavy chain is preferably a HLA-C class I heavy chain or a fragment thereof, more preferably HLA-Cw4.

Also in conformity with the invention, said compositions are advantageously combined with at least one pharmaceutically acceptable vehicle, preferably with a suitable carrier and/or an acceptable adjuvant, in particular the conventional adjuvants for human vaccines, such as, for instance alum.

Unexpectedly, compositions according to the invention are able to induce an immune response against HIV.

A preferred protocol of immunisation may be as follows:
- priming is preferably carried out either with an appropriate composition containing essentially at least an immunogenic fragment of HIV or a composition according to the invention, i.e.:
   . a composition essentially containing a multimolecular complex consisting essentially in at least an immunogenic fragment of the Env glycoprotein of an HIV and an HLA class I heavy chain (HC) or a fragment thereof, said elements being preferably non-covalently associated; or
   . a composition essentially containing particles containing, preferably at their surface, a multimolecular complex comprising essentially at least an immunogenic fragment of the Env glycoprotein of HIV and an HLA class I heavy chain or a fragment thereof;
- boosting is preferably carried out with a composition according to the invention, i.e. :
   . a composition essentially containing a multimolecular complex consisting essentially in at least an immunogenic fragment of the Env glycoprotein of an HIV and an HLA class I heavy chain (HC) or a fragment thereof, said elements being preferably non-covalently associated;
   . a composition essentially containing particles containing, preferably at their surface, a multimolecular complex comprising essentially at least an immunogenic fragment of the Env glycoprotein of HIV and an HLA class I heavy chain or a fragment thereof or
   . a composition containing a mixture of at least a fragment of HIV envelope glycoprotein and virion associated HLA class I heavy chains or a fragment thereof.

The subject of the present invention is also a method of preparation of a multimolecular complex according to the invention, said method comprising the following steps:
(a) transfecting an appropriate cell line with at least CD4, CXCR4 or CCR5 and HLA class I genes or fragments thereof,
(b) transfecting the cell obtained in (a) with a vector coding for at least a fragment of an HIV envelope glycoprotein, for instance gp120 or gp160 gene from any HIV and
(c) isolating the complex consisting of at least a fragment of HIV envelope glycoprotein and virion associated HLA class I heavy chains or a fragment thereof.

According to a first embodiment of said method, said non-human cell is selected in the group consisting in human or non-human cell lines, such as LCC.721.221, C8166, DAUDI or CHO cell lines.

According to another embodiment of said method, said isolating step is carried out by affinity chromatography.

The subject of the present invention is also another method of preparation of a multimolecular complex according to the invention, said method comprising the following steps:
(a) infecting an appropriate cell line with LAI virus or any HIV-1 or HIV-2 virus,
(b) lysing the infected cells and
(c) immunoprecipitating the obtained lysate with antibodies against at least an HIV envelope glycoprotein or a fragment thereof, for separating said multimolecular complex.

Apart from the foregoing arrangements, the invention also comprises other arrangements which will emerge from the description which follows, which refers to examples of implementation of the method which is the subject of the present invention.

It should nevertheless be clearly understood that these examples are given only by way of illustration of the subject of the invention and in no way constitute a limitation of the latter.

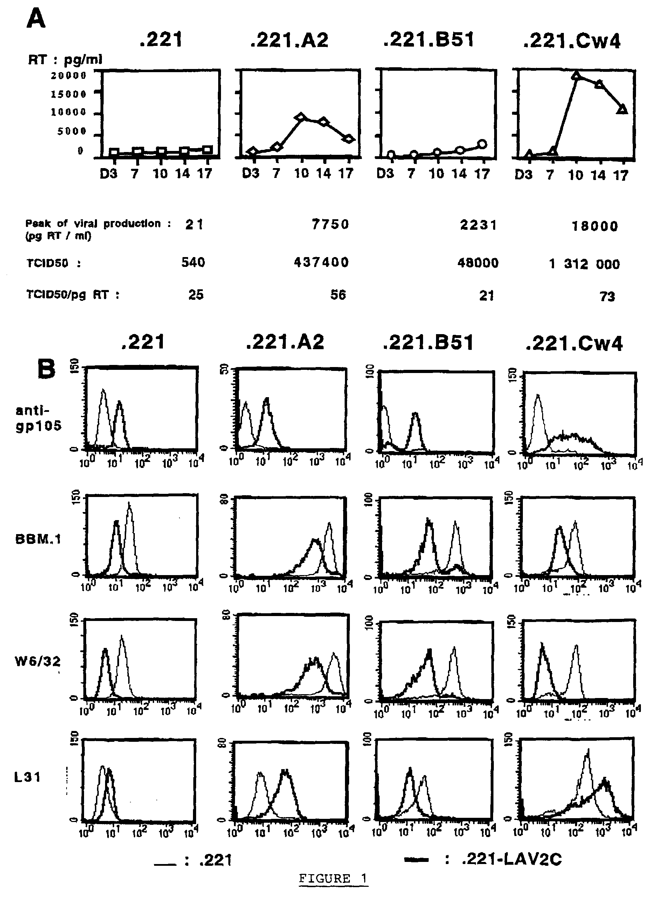

- Figure 1: Viral replication and surface modulation of HLA class I in .221 cells transfected with three different HLA class I HC genes (HLA-A2, HLA-B51 and HLA-Cw4); said figure illustrates the kinetic of viral replication (virus used was LAV-2/C) monitored by RT measurement and the determination of the infectivity titers of supernatants. Five x 10⁶ .221, 221.A2, .221.B51 or .221.Cw4 cells were infected with the HIV-2 CD4-independent virus LAV-2/C (100 pg equivalent RT/10⁶cells). After 1.5 hour at 37°C in a minimal volume, cells were washed 3 times, resuspended at 5.10⁶ cells/ml in RPMI-10% FCS. Figure 1A: Every 3 days, cells were passaged and supernatant assessed for RT production (Retrosys, Innovagen, Sweden) to establish the kinetic of infection. Supernatants collected at the peak of infection were titrated on the human T cell line C8166 to determine their 50% tissue culture infectivity dose (TCID₅₀). Cultures were routinely scored for the presence of syncitia. After syncitia disappearance, cells were washed and stained with an anti-HIV-2 gp105 rabbit serum (NIBSC, British MRC) and with the anti-HLA class I mAbs BBM.1, W6/32 and L31. Two x 10⁵ cells in FACS buffer (PBS-BSA 0,2%, sodium azide 0,1%) were distributed in V bottom 96 well trays on ice, incubated with the appropriate primary antibody for one hour at 4°C under agitation, washed 3 times in FACS buffer and incubated for 45′ at 4°C with the secondary Ab : swine anti-rabbit-FITC (Dako, Denmark) or Goat F(ab')2 anti-mouse IgG-PE (Immunotech, France). Cells were washed again 3 times, resuspended in paraformaldehyde 2% and then analysed on a FACSxalibur 4 colors instrument (Beckton Dickinson) using the Cell Quest software.
   Figure 1A illustrates that .221.Cw4 cells and .221.A2 cells are more permissive to viral replication than .221.B51 cells and produce more infectious viruses as demonstrated by the ratio between TCID₅₀ and the amount of RT. Figure 1B illustrates the expression of envelope antigen at the surface of infected cells and the down-modulation of HLA class I trimeric molecules recognised by the two monoclonal antibodies BBM1 and W6/32. More importantly, figure 1B illustrates the up-regulation of HLA class I HCs (recognised by mAb L31) and the two cell lines (.221.A2 and .221.Cw4) which sustain the strongest viral replication and produce the more infectious viruses.

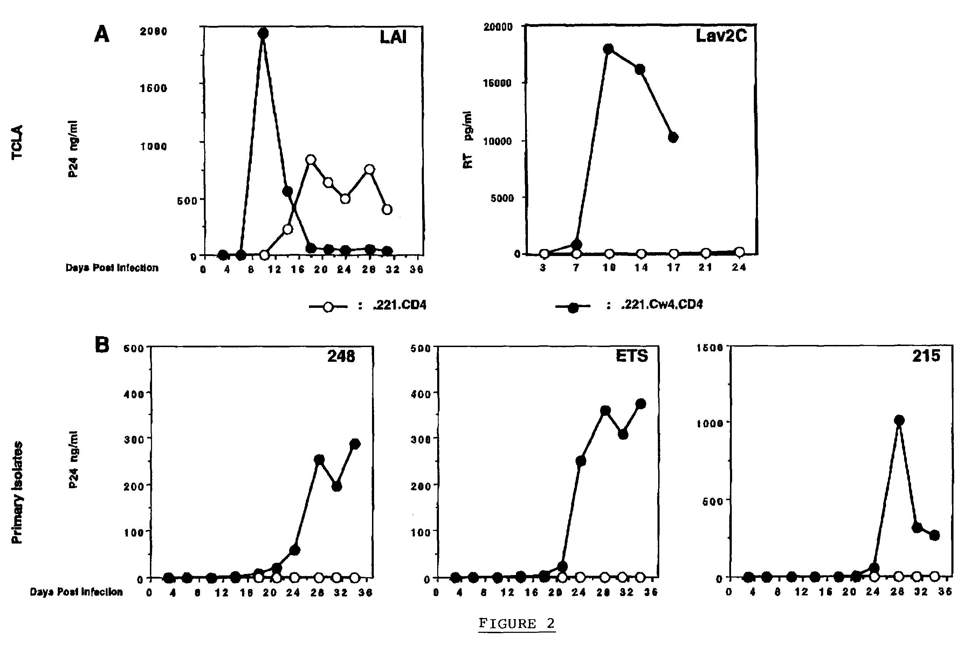

- Figure 2: Kinetic of infection of HIV-1 LAI, HIV-2 LAV-2/C and three primary X4 isolates on .221.CD4 and .221.Cw4.CD4 cells. Five x 10⁶ .221.CD4 or .221.Cw4.CD4 cells were infected as described in Fig 1 with either LAI, LAV-2/C, 215, 248 or ETS primary X4 isolates (2ng p24 equivalent/10⁶cells). The kinetic of infection was followed by measurement of HIV-1 p24 Ag (HIV-1 core profile ELISA, Du Pont de Nemours) or viral RT (LAV-2/C) as in Fig. 1. The different primary isolates 248, 215 and ETS were isolated from patient's PBMC by coculture with PHA-activated PBMC from healthy blood donors. Infected cells were grown in RPMI medium supplemented with 2 mM L-glutamine, 10% foetal calf serum, 500 IU/ml recombinant interleukin-2 (Proleukin, Chiron France, Suresnes), 1 IU/ml sheep polyclonal anti-interferon-α antibody (Valbiotech, Chatillon, France) 2 µg/ml polybrene (Sigma, St.Louis, MO) and antibiotics. For neutralisation experiments on PBMC, 125 nM of human recombinant chemokines RANTES (Biodesign International, Kennenbunk, ME, USA) or SDF-1α/BPSF (R&D Systems, Abingdon, UK) were added to the cultures at the time of infection and every three days when culture medium was replaced. The T cell line adapted (TCLA) strain HIV-1 LAI was propagated in the T cell line CEM or in .221.CD4 and .221.Cw4.CD4 cell lines.

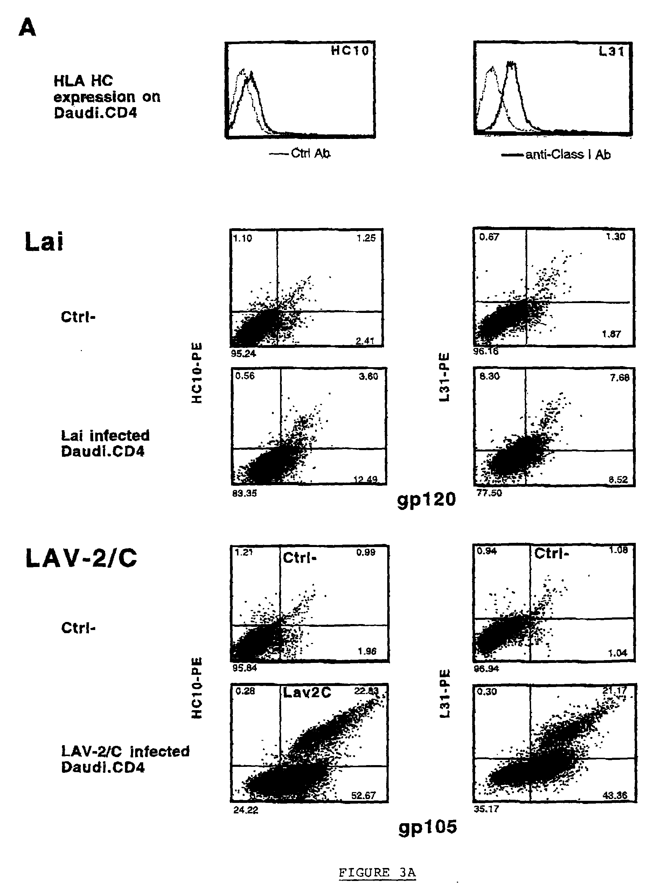

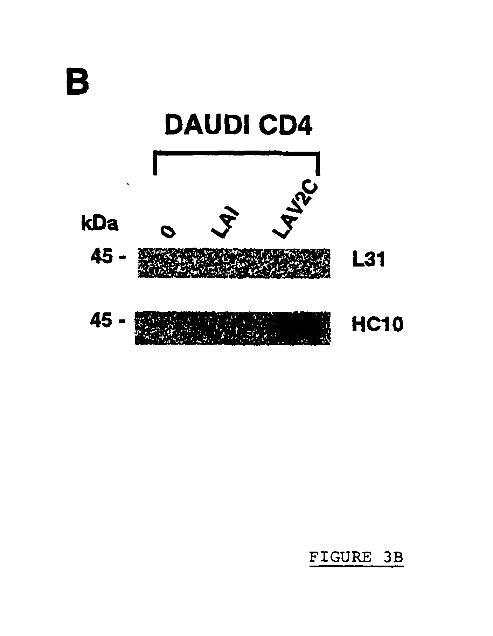

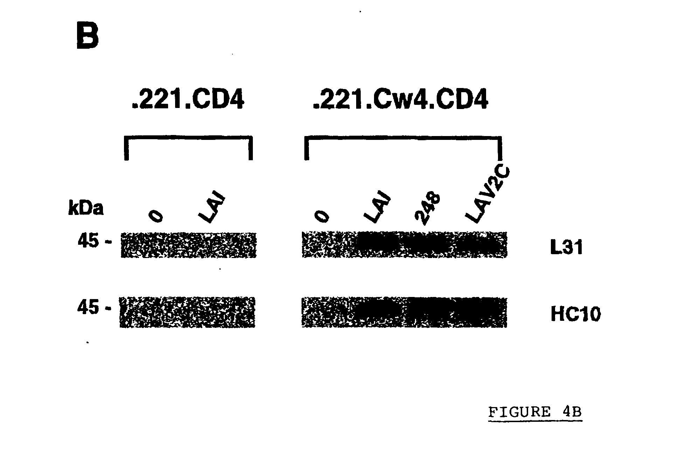

- Figure 3: figure 3A: Expression of HLA Class I heavy chains and Env at the surface of infected Daudi cells. Five x 10⁶ CD4-transfected Daudi cells (Daudi.CD4) were infected with HIV-1 LAI or HIV-2 LAV-2/C and stained with the anti-HLA class I HC mAbs L31 and HC10, and antisera specific for HIV-1 gp120 or HIV-2 gp105 as described in Fig.1. figure 3B: Western blot analysis of HLA class I heavy chains incorporated into LAI and LAV-2/C viruses grown on Daudi cells. LAI and LAV-2/C virus supernatants from Daudi.CD4 cells were centrifuged and treated as described in Fig.4B, hereafter for Western blot analysis with the two HLA class I heavy chains specific mAbs L31 and HC10. Supernatant from uninfected cells were used as control (0).

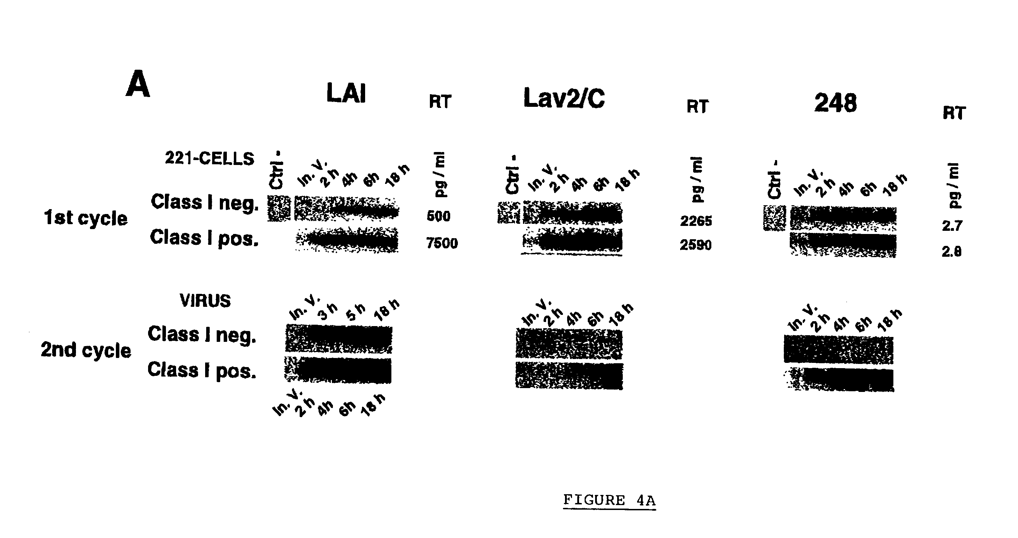

- Figure 4: PCR analysis of entry of LAI, LAV-2/C and 248 viruses in HLA class I positive or negative cells (1^{st} cycle) and entry of progeny viruses (2^{nd} cycle). Five x 10⁵ .221.CD4 or .221.Cw4.CD4 cells were incubated either 2 hours at 0°C (negative control, In.V.: Inactive virus), or 2, 4 ,6, 18 or 30 hours at 37°C with virus containing supernatant (MOI of 1 TCID₅₀/cell) previously treated with 20 µg/ml DNAse (Boehringer-Mannheim) in 10 mM MgCl₂, 30′ at 30°C. The viruses used in these experiments were : HIV-1 LAI, HIV-2 LAV-2/C or HIV-1 248 X4 primary isolate. After the first step of 2 hours incubation, cells were washed twice in serum-free medium, trypsinised (trypsine XIII, 15 µg/ml, Sigma) for 15′ at 4°C to remove any bound virus particles from the cell surface, washed again three times in complete medium and resuspended in 10% FCS-RPMI medium for a further incubation at 37°C. At each time point, (2, 4, 6 and 18 hours) an aliquot of 0.5.10⁶ cells from the assay was centrifuged and lysed as follows: after careful resuspension in 400 µl of Tris-KCl buffer (25 mM Tris-HCl pH 8, 125 mM KCl), 100 µl of NPK solution (1% NP40 plus 2.5 mg/ml proteinase K) (Boehringer-Mannheim) were added and the incubation done at 56°C for at least 3 hours. DNA samples were heated at 95°C for 15 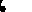 for DNAse inactivation. Twenty five µl of the lysate were used for the PCR reaction performed in a final volume of 50 µl containing 1.5 mM MgCl₂, 100 ng of each amplification primer, 0.2 mM of each dNTP and 2.5 U of *Taq* polymerase (Promega). Ctrl- was the negative control of the PCR amplification. The primers and probes used were R, sense primer, HIV-1 : 5'ggctaactagggaacccactg3' (LAI and 248), HIV-2 : 5'gaggctggcagattgagccctg3' (Lav2C); U5, anti-sense primer, HIV-1 : 5'ctgctagagattttccacactgac3', HIV-2 : 5'gcggcgactaggacagatggg3', and the Long Terminal Repeat LTR3 (hybridisation probe) HIV-1: 5'gtgtgtgcccgtctgttgtgtg3', HIV-2 : 5'ggccccacgcttgcttgctt3'. The PCR reaction was run in a Perkin-Elmer instrument as follows : 94°C, 2′, 1 cycle; 94°C,30˝- 58°C (HIV-1) or 60°C (HIV-2), 1′- 72°C, 1′, 35 cycles; 72°C, 7′, 1 cycle. All PCR reactions were separated by electrophoresis on a 2% agarose gel, transferred to a Gene Screen Plus membrane Amersham, UK (alkaline transfer) and the 195 bp products hybridised with their ³²P-labeled LTR specific probe. Supernatants from the 30 hours incubation were collected, centrifuged and filtered on a .45µm filter. An aliquot was centrifuged at 20 000 rpm for one hour and titrated out in a RT assay (Retrosys, Innovagen, Sweden). For each virus, equalised amounts of supernatants collected from .221.CD4 and .221.Cw4.CD4 cells were used in a second round of entry PCR on .221.Cw4.CD4 cells as described above. In addition, to further amplify the signal obtained, LAV-2/C PCR products were subjected to a nested PCR with the following set of primers : 5'-ggtagagcctgggtgttc-3', sens, and 5'-ggagagatgggagcacac-3', antisens. A photograph of the gel showing the amplified product of 139 bp long is presented on the LAV2/C bottom panel. Figure 4**B:** Western blot analysis of virion-associated HLA class I heavy chains. Equal amounts of LAI, LAV-2/C and 248 viruses generated on .221.Cw4.CD4 cells and LAI virus generated on .221.CD4 cells were pelleted by centrifugation (1h at 22.000 rpm) and resuspended in SDS-PAGE sample buffer [U.K. Laemmli, *Nature*, 1970, **227**, 680-5]. Proteins were resolved by SDS-PAGE (12.5% gel) and transferred to a nitrocellulose filter. Filters were blocked 16 hours at 4°C with 5% non-fat dry milk, 0.05% Tween 20 in PBS (PBS-T-milk) and then incubated with anti HLA class I mAbs (L31 or HC10) for 2h in the same blocking buffer. After washing with 0.05% Tween 20 in PBS (PBS-T), the filters were incubated with rabbit anti mouse-HRP complex (DAKO, Denmark) diluted 1/1000 in PBS-T-milk. After ten washes with PBS-T, peroxidase activity was revealed using an enhanced chemiluminescence system (ECL, Amersham, U.K.). As a negative control, an identical volume of supernatant from uninfected cells that was treated in the same way (lane 0) was used.

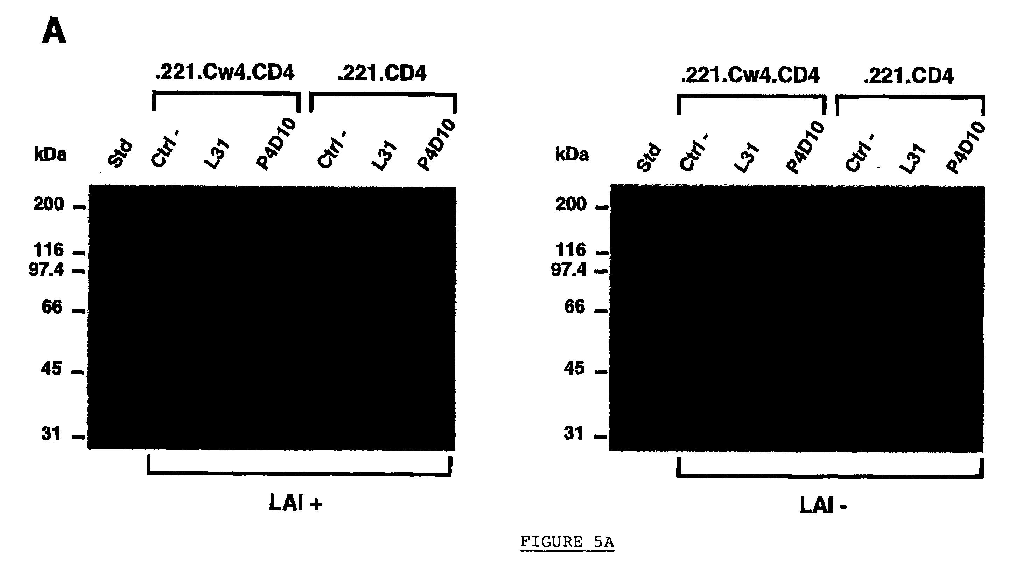

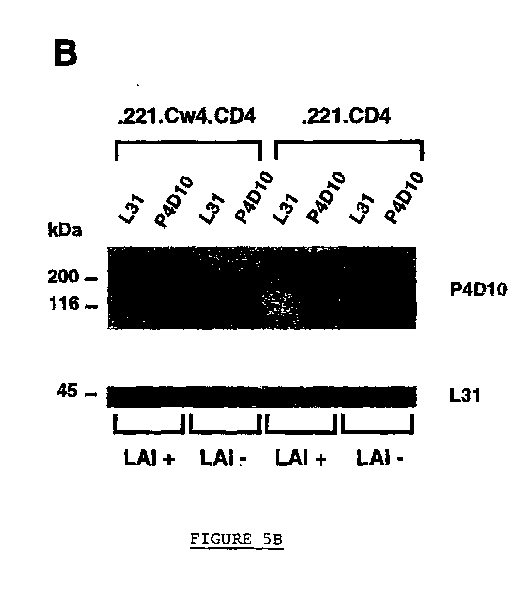

- Figure 5**A:** Co-precipitation of Env gp120/160 and HLA class I heavy chains from LAI infected .221.Cw4.CD4 cells. .221.Cw4.CD4 and .221.CD4 cells were infected with LAI as described in Fig.2 and surface biotinylated by addition of 0.15 mg/ml NHS-biotin (Pierce) to 1x10⁷ cells/ml in 0.1 M Hepes pH 8.0, 0.15 M NaCl for 30 min on ice. The reaction was quenched with 10 mM NH₄Cl and cells were washed and lysed in 0.5% NP40. Lysates were immunoprecipitated with an anti-HLA-C mAb (L31), an anti-LAI gp120 mAb (P4D10) and a control mAb using a procedure previously described [A. Cosma, *Anal. Biochem*., 1997, **252**, 10-4]. Immunoprecipitated material was resolved by SDS-PAGE (7.5% gel), proteins were transferred to nitrocellulose and probed with streptavidin-HRP for detection by ECL. Figure 5**B:** Western blot detection of HLA class I heavy chains co-precipitated with Env. NP40 lysates of LAI infected or uninfected .221.Cw4.CD4 and .221.CD4 cells were immunoprecipitated with L31 and P4D10 mAbs. After SDS-PAGE and transfer to nitrocellulose membrane the immunoprecipitated material was probed with mAb L31 to detect HLA class I HC and mAb P4D10 to detect LAI Env glycoproteins as described in Fig. 4B.

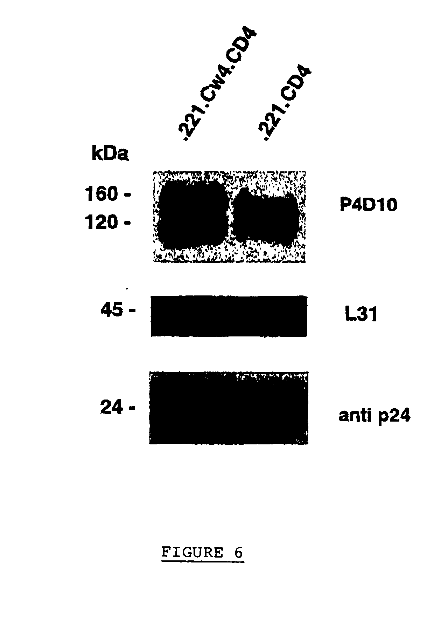

- Figure 6. Western blot analysis of Env gp120/160 glycoproteins incorporated into virions generated on .221.Cw4.CD4 and .221.CD4 cells. Equal amounts (50 ng of p24) of LAI grown on .221.Cw4.CD4 and .221.CD4 cells were treated like in Fig. 4B and the nitrocellulose filter probed with a pool of anti-p24 mAbs (clones EH12E1 and LH104-E from British MRC) to control the quantity of virus loaded on the gel, the anti-HLA class I HC mAb L31 to verify the presence of HLA class I and the anti-LAI gp120 mAb P4D10 to estimate the level of virion-associated Env glycoproteins.

### EXAMPLE 1: Modulation of cell surface expression of HLA class I on infected cells.

### Material and methods

### - Cell lines

. human cell lines (LCL.721.221) which lack chemical HLA class I HC A, B and C genes but express β₂m and become HLA class I surface positive upon transfection with HC genes [Shimizu, 1989] were used.
. cells transfected with HLA-Cw4 (.221.Cw4) [Biassoni, 1995], -A2 (.221.A2) and -B51 (.221.B51) and
   The cells were infected with the CD4-independent CXCR4 dependent TLCA virus, HIV-2 LAV2C.

### - Monoclonal antibodies

. The surface expression of HLA class I was investigated using a murine monoclonal antibody (L31) which had been obtained upon immunisation of mice with purified cell-free HIV-1 LAI virus and recognises HLA C-encoded HC in their β₂m free conformation [Beretta, 1987] [Grassi, 1991].
. Two additional antibodies, W6-32 and BBM1, specific for HLA class I trimeric complexes [Brodsky, 1979] and β₂m [Parham, 1983] respectively were also used as well as another anti-class I HC, HC10 (Stam, 1986).

Since L31 recognises, in addition to HLA-C some HLA-A and HLA-B isotypes, including A2 and B51 [Setini, 1996], cells transfected with HLA-Cw4 (.221.Cw4), -A2 (.221.A2) and -B51 (.221.B51) were used [Biassoni, 1995]. .221 cells are CD4 negative and express the HIV co-receptor CXCR4. Lav2C which is a CD4-independent, CXCR4-dependent HIV2 TCLA, was used in said experiments. In order to minimise parameters incidence others than HLA class I in Lav2C replication .221 transfected cells were subcloned and cells with similar amounts of HLA class II and equivalent amounts of CXCR4 were expanded for infection experiments. Non-transfected .221 cells were used as control.
- cell-surface is stained with the anti-HLA class I mAbs and an antiserum specific for HIV-2 viral envelope glycoprotein, a few days ofter the peak of viral replication corresponding to the disappearance of syncitia in culture. The kinetic of viral replication was monitored by RT measurement and the supernatants infectivity titers determined at the day of peak viral production.

### Results

As shown in Figure 1A, .221 and .221.B51 cells are only marginally permissive to the replication of LAV2/C. In contrast, LAV2/C replicated efficiently on .221.A2 and .221.Cw4. In addition, there were significative differences between the infectivity titers of cell-free supernatants (figure 1A): supernatants from .221.Cw4 cells and .221.A2 cells displayed a higher TCID₅₀ compared to supernatants from .221 and .221.B51 cells. The comparison of the TCID₅₀/ratios of each virus supernatant revealed that .221.Cw4 cells generated the most infectious viruses. All cell lines were surface positive for the viral envelope antigen gp105 (90 to 100 % fluorescent cells). Maximum Env expression was seen on .221.Cw4 cells. The trimeric HLA class I complexes recognised by BBM1 and W6-32 were down-modulated in the four cell lines, including .221, which expresses non-classical HLA class I antigens (Shimizu Y et al., 1989)decreases upon infection on -A2, -B51 as well as -Cw4 transfected cells. In contrast, there is a significant up-modulation of L31-reactive molecules on .221.Cw4 and .221.A2 but not .221.B51 cells.

Some level of viral replication occurs also on HLA class I negative cells and lack of HLA class I does not prevent the expression of viral protein at the cell surface (figure 1B) but rather results in a severe reduction of both the quantity of viruses produced and their infectivity.

Viral replication in the three cell lines varied significantly: it was maximal on .221.Cw4 cells, high on .221.A2 and low on .221.B51 cells (Figure 1b). Therefore, the increased surface expression of L31-reactive HLA class I HC apparently correlated to the extent of viral replication and to the infectivity of the viruses generated on the three cell lines indicating an association of these molecules with infectious viral particles.

In addition, there were evidences that viral replication could be influenced by the transfected HLA class I allele since .221.Cw4 cells were more permissive to LAV-2C replication than .221.A2 and much more than .221.B51 cells.

### EXAMPLE 2: Permissiveness of HLA class I HC positive and negative cells to the replication of TCLA and primary X4 viruses.

### Material and methods

### - cell lines

. cells transfected with HLA-Cw4 (.221.Cw4) [Biassoni, 1995], HLA-Cw4 and CD4 (.221.Cw4.CD4)

### Results

The results correspond to the evaluation of the permissiveness of HLA class I HC positive and negative cells to the replication of viruses other than LAV-2C.

The kinetic of replication of a CD4-dependent TCLA virus (HIV-1 LAI) and three primary HIV-1 isolates which use CXCR4 co-receptor was analysed. LAI was used as a control of X4 dependent virus. Primary isolates (215, 248 and ETS) were chosen on the basis of the capacity to replicate in CXCR4 expressing cell lines (MT2 and C8166) and of being inhibited by SDF-1α (the natural ligand of CXCR4) (E. Oberlin et al., *Nature*, 1996, **382**, 833-5) in PBMC infection assays: LAI (100% of inhibition), ETS (93% of inhibition), 215 (63% of inhibition) and 248 (45% of inhibition).

.221.Cw4 cells were used since they showed the highest permissivity to LAV-2C, .221 and .221.Cw4 cells were transfected with the human CD4 gene and tested for their capacity to support the replication of LAI and the three primary isolates replication. As shown in Figure 2A, LAI was able to infect both .221.CD4 and.221.Cw4.CD4 cells, although the kinetic of replication was delayed in the absence of HLA class I negative cells. In contrast, LAV-2C failed to replicate on .221.CD4 cells but replicated on .221.Cw4.CD4 cells.

The three isolates tested failed to replicate in the HLA class I negative .221.CD4 cells although they all replicated efficiently in the HLA class I positive cells .221.Cw4.CD4 (Figure 2B). Thus, the expression of HLA class I HC was found to be indispensable for the replication of HIV-1 primary X4 isolates and LAV2C. HIV-1 LAI differed from the other viruses since it could be propagated, although less efficiently, in the HLA class I negative cells.

These data are in apparent contradiction with previous reports concluding on the dispensability of HLA class I for virus replication which were based on the permissivity of HLA class I surface negative cells, DAUDI, to HIV infection [Clapham, 1991]. It has been reported that LAI virus replicates on DAUDI cells and does not incorporated HLA class I molecules on HIV-LAI produced by DAUDI cells [Le Gall, 1997].

DAUDI cells exhibit a genetic defect in the β₂m gene, but they express high intracellular levels of HLA class I HC transcripts [Ploegh, 1979].

Their permissivity to viral replication may be restricted to some TCLA viruses, like LAI, which are less dependent on the presence of HLA class I for replication. Alternatively, virus incorporation of HLA class I could occur even in the absence of a functional β₂m gene by recruitment of the intracellular pool of HLA class I HC.

### EXAMPLE 3: Expression of HLA class I HC and viral envelope glycoproteins on CD4-transfected DAUDI cells, infected with LAV2C or LAI.

### Material and methods

### - Cell lines

Daudi cells (Le Gall, 1997)
Daudi-CD4 cells

### - monoclonal antibodies (mAbs)

Two HLA class I HC specific mAbs, L31 and HC10 [Stam, 1986] were used and antisera specific for HIV-1 or HIV-2 Env.

### Results

Uninfected DAUDI-CD4 cells were weakly stained by the two anti-HLA class I HC mAbs (Figure 3A). Infection with LAV2C (Figure 3A) but not LAI (Figure 3A) induced a marked increase in the L31 (Figure 3) and HC10 fluorescence (Figure 3A) which was evident on a population of Env-high and HLA class I HC-high cells indicating the association of HLA class I HC with LAV-2C viral particles.

### EXAMPLE 4: Western blot analysis on purified LAV2C and LAI viral particles generated in DAUDI-CD4 cells.

The two HC specific mAbs are used (L31 and HC10) (Figure 3B).

A 45 kDa band corresponding to HLA class I HC within LAV2C but not LAI viral lysates was detected. The protein was absent in the control supernatant from uninfected cells.

The protein was absent in control supernatants from uninfected cells. Of the two HLA class I HC mAbs used, HC10, but not L31, stained the 45 kD band. This was unexpected since the surface reactivity of both mAbs on LAV2/C infected cells was similar (Figure 3A). The reactivity of the two mAbs for native versus denatured determinants may account for these differences. The absence of HLA class I HC associated to LAI virus grown on DAUDI-CD4 cells was in agreement with results previously published by other authors (Le Gall, 1997).

These data showed that:
i) DAUDI cells cannot be considered as *bona fide* HLA class I negative cells since they express low levels of HLA class I HC at the cell surface and become intensely positive following infection with LAV2C,
ii) HLA class I HC can be incorporated within viral particles even in the absence of β₂m,
iii) there is a clear association between the capacity of a given virus (LAV2C versus LAI) to incorporate HLA class I HC and to induce their cell-surface expression,
iv) TCLA viruses differ in their dependency towards class I HC.

These data also bring further support to the conclusion that, on infected cells, surface HLA class I HC are, at least in part, associated to infective viral particles as also suggested by the data obtained on .221.Cw4 and A2 cells (Figure 1).

### EXAMPLE 5 : PCR analysis of entry of LAI, LAV-2/C and 248 viruses in HLA class I positive or negative cells (1^{st} cycle) and entry of progeny viruses (2^{nd} cycle).

To determine whether viral entry was hindered in Class I negative cells, the ability of .221.CD4 and .221.Cw4.CD4 cells to support a single cycle of viral replication was compared using an entry PCR assay [Zack, 1990]. Cells were infected with equal amounts of either LAI, LAV-2/C or one of the primary isolates, HIV-1 248, and the accumulation of strong stop cDNA was measured over 18 hours. As shown in Fig.4A, entry was achieved by all three viruses, although there was a significant delay in the accumulation of cDNA in the HLA class I negative cells. The delay was more pronounced with LAI and was associated with lower viral RT production after 30 hours. Viral RT production in LAV-2/C and 248 infected .221.CD4 and .221.Cw4.CD4 cells was identical, indicating there was no post-entry blockade in .221.CD4 cells.

To determine whether HLA class I molecules acquired by virions after the first cycle of replication influence viral entry, viruses generated from .221.CD4 and .221.Cw4.CD4 cells were tested in a second cycle of replication in .221.Cw4.CD4 cells. The results showed distinct patterns of virus entry for HLA class I positive and negative cell-derived viruses (Fig.4A). LAI generated on .221.CD4 cells displayed delayed entry kinetics and a decreased level of strong stop cDNA 18 hours post-infection compared to LAI generated on .221.Cw4.CD4 cells. The HLA class I negative-cell derived LAV2/C and 248 viruses showed no detectable accumulation of strong stop cDNA, indicating a defect in a pre-retrotranscription step. However, when generated on HLA class I positive cells, the same viruses were entry competent.

To confirm the presence of HLA class I heavy chains on the viruses generated from .221.Cw4.CD4 cells, Western blot assays of the concentrated viral lysates were performed using HLA class I heavy chain specific mAb L31 and another heavy chain mAb HC10. LAI, LAV-2/C and 248 viruses expanded on .221.Cw4.CD4 cells were tested. As a control HLA negative virus, LAI generated from .221.CD4 cells was tested, since it could not be poossible to generate sufficient amounts of HLA class I negative LAV-2/C and primary isolates for biochemical analysis. All viruses obtained from .221.Cw4.CD4 cells, including LAI, displayed a 45 kDa band reactive with both heavy chain specific mAbs (Figure 4B) which was absent on LAI grown on the HLA class I negative cells.

These data showed that lack of virion-associated HLA class I results in the generation of virions whose competence to enter cells is impaired. No major blockade at the post-entry level occurs on HLA class I negative cells as indicated by the levels of viral RT produced during the first cycle of replication. This is also supported by the finding that LAI can efficiently replicate in .221.CD4 cells, even if at a lower rate than in .221.Cw4.CD4 cells. The delayed kinetics of entry into .221.CD4. cells during the first cycle PCR could be explained by the lower level of CD4 expressed by these cells. However, LAV-2/C, which is CD4 independent, was also delayed indicating that other factors may be involved.

Said incorporation is required for the acquisition of the competence to enter cells.

The two TCLA viruses LAI and LAV2C, and two primary X4 isolates (248 and 215) were used to infect .221.Cw4 and .221.Cw4.CD4 cells and, after one single cycle of replication, were concentrated by ultracentrifugation and quantified in a RT assay.

This conclusion is also supported by the comparison of the infectivity titers of LAI viruses generated on HLA class I negative and positive cells. LAI cell-free supernatants from .221.CD4 and .221.Cw4.CD4. Infected cells were collected at the peak of infection and titrated on PHA-activated peripheral blood mononuclear cells. The infectivity titers were measured at day 6. There was a significative difference between LAI viruses propagated on the two cell lines (13 versus 93 TCID₅₀/ngp24 for LAI viruses propagated on .221.CD4 and 221.Cw4.CD4 cells respectively) indicating that HIV-1 LAI acquires a higher infectivity upon replication in HLA class I positive cells.

Table I illustrates the fact that viruses grown on HLA class I positive cells are more infectious than viruses grown on HLA class I negative cells.

**TABLE I**

| | p24 ng/ml | RT cpm : 10-3/ml | Infectivity TCID50/ngp24 |
|---|---|---|---|
| LAI Class I Negative | 374 | 1570 | 9 |
| LAI Class I Positive | 826 | 1644 | 68 |

### EXAMPLE 6: Co-precipitation of Env gp120/160 and HLA class I heavy chains from LAI infected .221.Cw4.CD4 cells.

Since flow-cytometry data indicated the co-expression of Env and HLA class I HC (see figure 1B and figure 3A) at the membrane of infected cells, and the association of their surface expression with the production of infective viral particles, the presence of Env/HLA class I complexes was tested on LAI-infected .221.CD4 and .221.Cw4.CD4 cells.

Cells were surface biotinylated and the cell lysates immunoprecipitated with either L31 or an anti-LAI-gp120 mAb (P4D10) (Figure 5A). Immunoprecipitation of LAI infected .221.CD4 cells with the anti-gp120 mAb P4D10 revealed, in addition to gp120 and gp160, two major bands of an apparent molecular weight corresponding to CD4 (58 kDa) and CXCR4 (48 kDa). The P4D10 immunoprecipitates of LAI infected .221.Cw4.CD4 cells exhibited an additional band of 45 kDa whose molecular weight corresponded to that of the band precipitated by L31 on both infected and non-infected cells. A weak band of an apparent molecular weight of 160 was evidenced in the L31 immunoprecipitate of .221.Cw4.CD4 LAI infected cells. To assess the identity of the 45 kDa band with HLA class I HC, the same experiment was performed without biotin surface labelling and we probed the nitrocellulose filter with P4D10 and L31 (Figure 5B). The 45 kDa band precipitated by the anti-gp120 mAb P4D10 on infected .221.Cw4.CD4 cells was stained by L31. This band was absent in both the infected and non infected .221.CD4 cells as well as in the P4D10 immunoprecipitates of uninfected .221.Cw4.CD4 cells. Similar data were obtained with LAV2C-infected .221.Cw4 cells. Thus, a specific interaction between the HIV envelope glycoprotein and the HLA class I HC occurs in infected cells.

### EXAMPLE 7: Western blot analysis of Env gp120/160 glycoproteins incorporated into virions generated on .221.Cw4.CD4 and .221.CD4 cells.

To test whether the pattern of Env expression by mature viral particles was influenced by the presence or absence of virion-associated HLA class I HC, a Western blot analysis of Env glycoproteins from purified HLA class I positive or negative LAI viral particles was performed. The Env content of the HLA class I positive virus, as estimated by densitometry analysis, was 4 folds higher compared to that of HLA class I negative virus (Figure 6). The amount of p24 in virus preparations from .221.CD4 and .221.Cw4.CD4 was equivalent.

This experiment could only be performed on HLA class I negative and positive LAI viruses since we could not propagate the other viruses on the HLA class I negative cells. Although not strictly dependent on HLA class I for replication, LAI incorporates HLA class I HC when grown on .221.Cw4 cells and acquires a higher infectivity. Thus, the difference in Env expression by HLA class I negative and positive LAI viral particles probably reflects a general phenomenon associated to the HLA class I-dependent acquisition or increase in infectivity. Such difference may be due to reduced shedding or to enhanced incorporation of Env glycoproteins within nascent HLA class I positive viral particles.

### EXAMPLE 8: Immunoprotective role of the instant compositions.

Unexpectedly, the instant compositions given according to an appropriate protocol, preferably during the boosting step, induce a immune protection against HIV infection.

Primary and TCLA viruses display significative differences in their susceptibility to neutralisation by anti-Env antibodies. The need for HLA class I HC incorporation for the acquisition of entry-competence and the presence of an Env/HLA class I complex on infected cells suggest that the expression of a functional viral envelope requires the formation of such a complex. The expression of Env determinants involved in the interactions with CD4 and the co-receptors may be different in HLA negative or positive viruses as a consequence of Env SU conformational modifications induced by the association with HLA class I HC.

Therefore, in order to induce antibodies to the relevant Env determinants, it is preferable to use compositions according to the invention comprising Env/HLA class I antigens rather than isolated Env antigens.

### EXAMPLE 9: Preparation of a composition containing gp120 of HIV-1 and HLA-C class I heavy chain.

.221.Cw4.CD4 cells are infected with LAI virus. The kinetic of viral antigen measurement. A replication is followed by p24. At the day of peak viral replication, cells are washed, and lysed in 0.5 % NP40 containing protease inhibitors (aprotinine and PMSF, SIGMA, St Louis, USA). The lysates are immunoprecipitated with an anti―LAI gp120 mAb (mAb P4D10, for instance (L. Akerblom et al., *AIDS*, 1990, **4**, 953-960)), using the following procedure:

Protein A-Sepharose (10 µl) (Pharmacia) is incubated with 5 µg of rabbit anti-mous IgG (RaMIgG) Dako) plus 200 µl of incubation buffer at 4°C on rotation for one hour, and then free antibody is washed away with cold PBS. Successively the protein A-Sepharose:RAMIgG complex is incubated with 5 µg of P4D10 mAb in the same condition as described above. The cell lysate is incubated with protein A-Sepharose:RAMIgG:P4D10 for 3 hours at 4°C on rotation. Protein A-Sepharose:antibodies complex is then washed seven times with cold "washing buffer with protein" and three times with cold "washing buffer without protein".

The quality control analysis of the immunoprecipitated material is performed by Western blot with mAb L31 and P4D10 on nitrocellulose filters after transfer of the proteins resolved in SDS-PAGE (figure 5B).

### EXAMPLE 10: Preparation of recombinant attenuated viral particles containing a complex gp160 (or gp120) and HLA-Cw4 class I antigen.

The recombinant MVA vaccinia virus is, for instance as described in US Patent 5 185 146.

The sequence coding for the gp160 or the gp120 is, for instance as described in International PCT Application WO 86/02383 ; M. Alizon et al., *Nature*, 1984, 312, 757-760 ; M. Alizon et al., *Cell*, 1986, **46**, 63-74; D. Klatzmann et al., *Nature*, 1984, **312**, 767-768 or L.O. Arthur et al., *Proc. Natl. Acad. Sci. USA*, 1987, **84**, 8583-8587.

The sequence coding for the HLA-Cw4 class I antigen is, for instance described in Grassi, 1991.

### REFERENCES

- P.J. Bjorkman et al., *Annu. Rev. Biochem*., 1990, **59**, 253-88 ;
- E. Schnabl et al., *J. Exp. Med*., 1990, **171**, 1431-42 ;
- J. Zemmour et al., *J. Exp. Med*., 1992, **176**, 937-50 ;
- O. Schwartz et al., *Nat. Med*., 1996, **2**, 338-42 ;
- K.L. Collins et al., *Nature*, 1998, **391**, 397-401 ;
- S. Le Gall et al., *Immunity*, 1998, **8**, 483-95 ;
- Y. Shimizu et al., *J. Immunol*., 1989, **142**, 3320-8 ;
- A. Beretta et al., *Eur J. Immunol.*, 1987, **17**, 1793-8 ;
- F. Grassi et al., *J. Exp. Med*., 1991, **174**, 53-62 ;
- F.M. Brodsky et al., *Immunol. Rev*., 1979, **47**, 3-61 ;
- P. Parham et al., *J. Biol Chem*., 1983, **258**, 6179-86 ;
- N.J. Stam et al., *J. Immunol*., 1986, **137**, 2299-306 ;
- A. Setini et al., *Hum. Immunol*., 1996, **46**, 69-81 ;
- R. Biassoni et al., *J. Exp. Med*., 1995, **182**, 605-9 ,
- E. Oberlin et al., *Nature*, 1996, **382**, 833-5 ;
- J.A. Zack et al., *Cell*, 1990, **61**, 213-22 ;
- P.R. Clapham et al., *Virology*, 1991, **181**, 703-15 ;
- S. Le Gall et al., *Virology*, 1997, **229**, 295-301 ;
- H.L. Ploegh et al., *Proc. Natl. Acad. Sci. USA*, 1979, **76**, 2273-7 ;
- C.D. Rizzuto et al., *J. Virol*, 1997, **71**, 4847-51 ;
- R. Cantin et al., *J Virol*., 1997, **71**, 1922-30 ;
- L.O. Arthur et al., *Science*, 1992, **258**, 1935-8.

As is apparent from the foregoing description, the invention is in no way limited to those modes of execution, embodiments and modes of application which have now been described more explicitly; on the contrary, it encompasses all the variants thereof which may occur to those skilled in the art, without deviating from the framework or the scope of the present invention.

## Claims

1. An immunogenic composition, characterised in that it essentially contains a multimolecular complex consisting essentially in at least an immunogenic fragment of the Env glycoprotein of an HIV and an HLA class I heavy chain HC) or fragments thereof.

2. An immunogenic composition, characterised in that it essentially contains particles containing preferably at their surface, a multimolecular complex comprising essentially at least an immunogenic fragment of the Env glycoprotein of HIV and an HLA class I heavy chain or a fragment thereof.

3. The immunogenic composition according to claim 2, characterised in that said particles are selected in the group consisting in viral particles and transfected cells.

4. The immunogenic composition according to claim 2 or to claim 3, characterised in that said viral particles are preferably selected in the group consisting of attenuated viruses, recombinant attenuated viral particles and purified pseudoparticles.

5. The immunogenic composition according to claim 3 or to claim 4, characterised in that said viral particles are preferably selected among MVA vaccinia virus, adenovirus, canarypox, BCG, poliovirus, chickenpox, smallpox or baculovirus.

6. The immunogenic composition according to claims 2 to 5, characterised in that said viral particles further contain at least a fragment of HIV Gag or Pol protein.

7. The immunogenic composition according to claim 2 or to claim 3, characterised in that said transfected cells are preferably selected in the group consisting in human or non-human cells, more preferably in the group consisting in B cells and CHO cells.

8. An immunogenic composition, characterised in that it essentially contains a mixture of at least a fragment of HIV envelope glycoprotein and virion associated HLA class I heavy chains or a fragment thereof.

9. The immunogenic composition according to claim 1 to 8, characterised in that said HIV Env glycoprotein is selected in the group consisting of gp120, gp160 or immunogenic fragments thereof either from HIV-1 or HIV-2.

10. The immunogenic composition according to claim 1 to 9, characterised in that said HLA class I heavy chain is preferably a HLA-C class I heavy chain, more preferably HLA-Cw4 or a fragment thereof.

11. The immunogenic composition according to claim 1 to 10, characterised in that it is combined with at least one pharmaceutically acceptable vehicle.

12. The immunogenic composition according to claim 11, characterised in that it is advantageously combined with a suitable carrier and/or an acceptable adjuvant, in particular the conventional adjuvants for human vaccines, such as, for instance alum.

13. A method of preparation of a multimolecular complex according to claim 1, characterised in that it comprises the following steps:
(a) transfecting an appropriate cell line with at least CD4, CXCR4 or CCR5 and HLA class I genes or a fragment thereof,
(b) transfecting the cell obtained in (a) with a vector coding for at least a fragment of an HIV envelope glycoprotein from any HIV, and
(c) isolating the complex consisting of at least a fragment of HIV envelope glycoprotein and virion associated HLA class I heavy chains or a fragment thereof.

14. A method of preparation of a multimolecular complex according to claim 13, characterised in that said cell lines are selected in the group consisting in human or non-human cell lines, such as LCC.721.221, C8166, DAUDI or CHO cell lines.

15. A method of preparation of a multimolecular complex according to claim 13 or claim 14, characterised in that said isolating step is carried out by affinity chromatography.

16. A method of preparation of a multimolecular complex according to claim 1, characterised in that it comprises the following steps:
(a) infecting an appropriate cell line with LAI virus or any HIV-1 or HIV-2 virus,
(b) lysing the infected cells and
(c) immunoprecipitating the obtained lysate with antibodies against at least an HIV envelope glycoprotein or a fragment thereof, for separating said multimolecular complex.
